Europäisches Patentamt

European Patent Office

Office européen des brevets

(11) Publication number: **0 108 919**
**B1**

# EUROPEAN PATENT SPECIFICATION

(45) Date of publication of patent specification: **13.07.88**

(51) Int. Cl.⁴: **C 12 P 21/00** // C12N5/00

(21) Application number: **83110013.6**

(22) Date of filing: **06.10.83**

(54) Process for producing T cell-activating factor.

(30) Priority: **16.11.82 JP 199695/82**

(43) Date of publication of application:
**23.05.84 Bulletin 84/21**

(45) Publication of the grant of the patent:
**13.07.88 Bulletin 88/28**

(84) Designated Contracting States:
**DE FR GB**

(56) References cited:

CHEMICAL ABSTRACTS, vol. 97, no. 13, 27th
September 1982, page 180, no. 105362a,
Columbus, Ohio, US Y. OKAI et al.: "3T3
fibroblasts are stimulated by 12-0-
tetradecanoylphorbol 13-acetate to produce
thymocyte-activating factors"

The file contains technical information
submitted after the application was filed and
not included in this specification

(73) Proprietor: Okai, Yasuji
3-9-11 Naka-machi Machida-shi
Tokyo (JP)
(73) Proprietor: Yamashita, Uki
1-5302 Iseigaoka Yahatanishi-ku
Kitakyushu-shi Fukuoka-ken (JP)

(72) Inventor: Okai, Yasuji
3-9-11 Naka-machi Machida-shi
Tokyo (JP)
Inventor: Yamashita, Uki
1-5302 Iseigaoka Yahatanishi-ku
Kitakyushu-shi Fukuoka-ken (JP)

(74) Representative: Vossius & Partner
Siebertstrasse 4 P.O. Box 86 07 67
D-8000 München 86 (DE)

(56) References cited:
CHEMICAL ABSTRACTS, vol. 85, no. 9, 30th
August 1976, page 416, no. 61100j, Columbus,
Ohio, US J.C. BARTHOLOMEW et al.: "Effect of
serum on the growth of Balb 3T3 A31 mouse
fibroblasts and an SV40-transformed
derivative"

Courier Press, Leamington Spa, England.

# 0 108 919

## Description

The present invention relates to a process of producing a T cell-activating factor. More specifically, the present invention relates to a process for producing a T cell-activating factor which comprises culturing human fibroblast cells in a medium containing a phorbol, lectin, endotoxin or a virus, accumulating the T cell-activating factor in the medium and recovering the T cell-activating factor from the medium.

Heretofore, as therapy for diseases such as cancer and the like, various methods for activating the function of immunocytes of the patient have been tested. It has been reported that T cell-activating factors are very useful substances [Cheever M. A. *et al.*, J. Exp. Med. *155*, 968—980, 1982].

There are T cell-activating factors used interleukin L [Mizel S. B. *et al.*, J. Immunol., *120*, 1497—1503 (1978)], interleukin 2 [Gillis S. *et al.*, J. Immunol., *120*, 2027—2032 (1978)], etc. These T cell-activating factors have been prepared using lymphocytes or mice, rats or humans or spleen cells of mice or rats. However, the methods are insufficient in that the products are produced only in a small amount.

The in vitro production of thermocyte-activating factors using 12-0-tetradecanoylphorbol-13-acetate stimulated mouse 3T3-fibroblasts is known from Y. Chai et al. (Febs Letters *142* (1982), 93—95; Chem. Abstr. *97* (1982), 180).

The present inventors have studied to produce T cell-activating factors and found that a large amount of T cell-activating factors can be produced by culturing human fibroblasts.

According to the present invention, a T cell-activating factor is produced by culturing human fibroblast cells in a medium containing phorbols, lectins, endotoxins or viruses, accumulating the T cell-activating factor in a medium and recovering it therefrom.

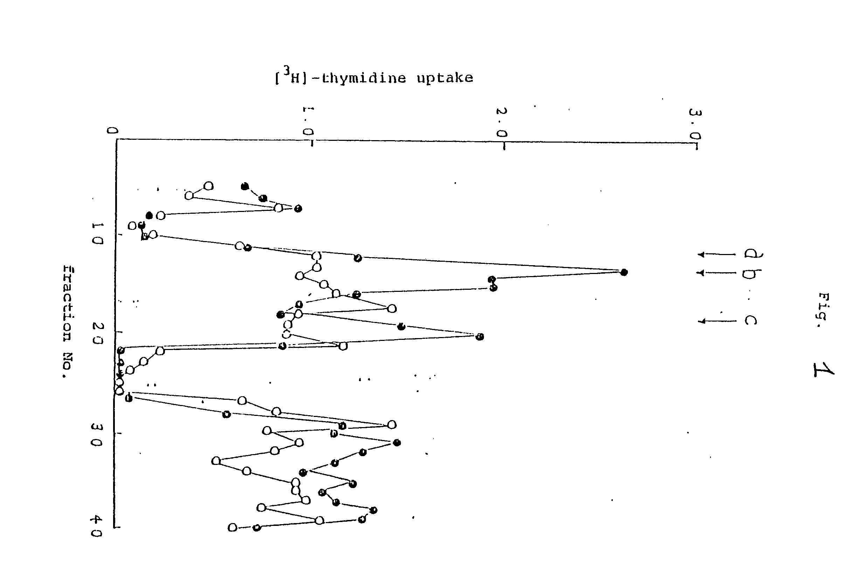

Fig. 1 shows Sephadex G-100 column chromatography of the T cell-activating factor produced by fetal human fibroblast cells. In the drawing, ●—● refers to the presence of TPA; o—o refers to the absence of TPA; a refers to bovine serum albumin (molecular weight: 87 Kd); b refers to horse radish peroxidase (molecular weight: 40 Kd); and c refers to cytochrome C (molecular weight: 13,000 Kd).

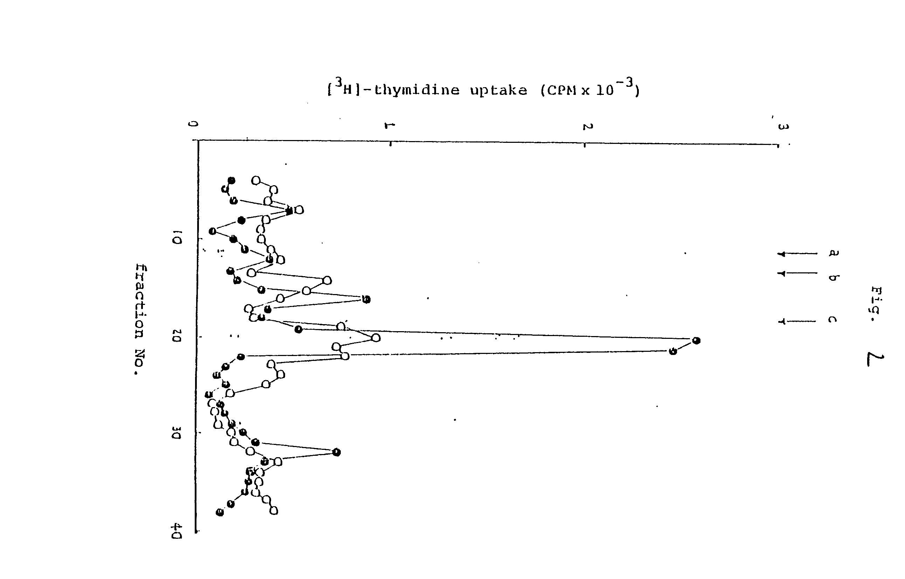

Fig. 2 shows Sephadex G-100 column chromatography of the T cell-activating factor produced by SV40 infected fetal fibroblast cells. In the drawing, ●—● refers to the presence of SV40, o—o refers to the absence of SV40 and a, b and c have the same meanings as those in Fig. 1.

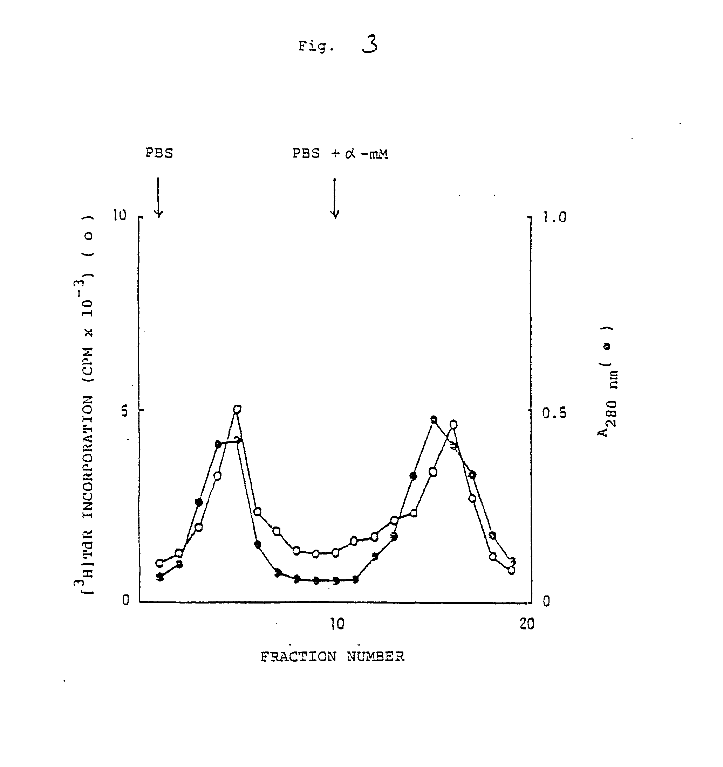

Fig. 3 illustrates Concanavalin A Sepharose chromatography of T cell-activating factors at 10 Kd of Sephadex G-100 chromatography. Open and closed circles show the activities of T cell DNA synthesis and absorbance at 280 nm. Arrows in the figure represent the positions of the applied buffer, PBS and PBS containing α-methyl mannoside.

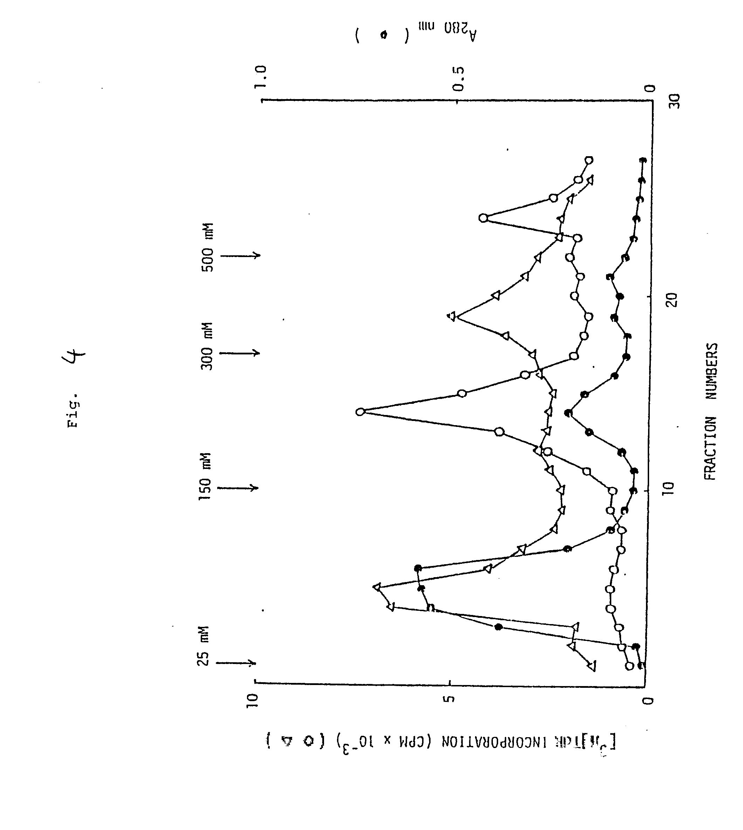

Fig. 4 illustrates CM Sephadex chromatography of 10 Kd factors produced by SV40-transformed human fibroblasts. Open circles and triangles show the activities of DNA synthetic response of T cells and 3T3 fibroblasts. Arrows in the figure represent the applied positions of the different elution buffer.

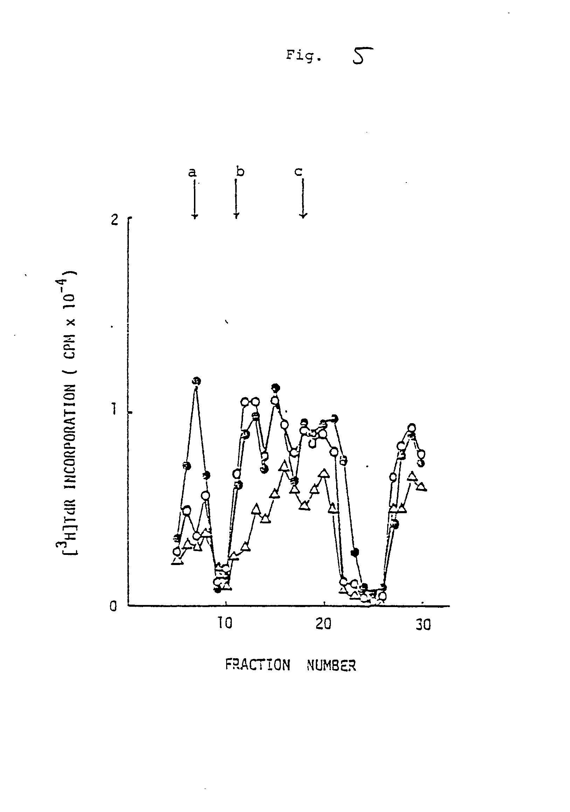

Fig. 5 illustrates Sephadex G-100 column chromatography of the mixture of TPA and the conditioned medium of normal human fibroblasts. Open and closed circles show the activities of normal and TPA-containing medium. Triangles represent the background activities of the fresh medium. Arrows in the figure show the same markers as in Fig. 1.

Human fetal fibroblasts are preferably used in the present invention.

As the medium used for the cultivation of fibroblasts, a medium conventionally used for the cultivation of animal cells is used. For example, RPMI1640 medium (product of Grand Island Biological Co. —Gibco) containing 1—10% fatal calf serum, and MEM medium (product of Nissui Seiyaku Co.) containing 1—10% fetal calf serum are preferably used.

The composition of RPMI1640 and MEM medium are described in "Tissue Culture" edited by Junnosuke Nakai *et al.*, Asakura Shoten, p. 9—11, 1976.

As the phorbols, phorbol, phorbol myristate, phorbol-13-acetate and phorbol myristic acetate [referred to as PNA hereinafter, synonym of 12-O-tetradecanoylphorbol-13-acetate [referred to as TPA hereinafter)] are preferably used.

As the lectins, phytohemagglutinin and concanavalin A (ConA) are used.

As the endotoxins, lipopolysaccharide and purified protein derivatives are used.

As the viruses, cancer viruses such as SV-40 and Kirsten murine sarcoma virus are used.

Phorbols are used generally in a concentration of 1—1000 ng, preferably 10—100 ng per 1 ml of the medium.

Viruses are used in a ratio of 10—1000, preferably about 100 per cell.

Culturing is carried out in a liquid under aeration according to conventional culturing conditions for animal cells. Aeration is carried out with 1—10% $CO_2$ and 90—99% air. The temperature for culturing is 25—40°C, preferably 30—39°C, and the pH is 7.5—8.3, preferably 7.8—8.0. After culturing for 8—12 hours in the case of using phorbols and 2—4 days after infection in the case of using viruses, the culture medium is recovered.

Recovery of T cell-activating factors from the culture medium is carried out by the method generally used for recovering proteins from the culture medium of animal cells. That, is the culture medium is subjected to decantation or centrifugation to remove cells. The thus obtained supernatant fluid is concentrated under reduced pressure and the concentrate is subjected to Sephadex G-100 column

2

chromatography. The resultant fractions are concentrated under reduced pressure to recover T cell-activating factors.

Since ths T cell-activating factors of the present invention activate the DNA synthesis of T lymphocytes, they are expected as the therapeutic agent for the diseases such as cancer.

The activity of T cell-activating factor is determined in the following manner. $2.5 \times 10^5$ cells of thymocytes of C3H/He mouse [product of Seiwa Jikken Dobutsu Co] are suspended in 0.1 ml of RPMI1640—10% fetal calf serum. To the suspension is added 50 or 100 µl of the supernatant of the culture medium of fibroblast cells or of each fraction of column chromatography. 2.5 µg/ml ConA is added to the mixture and the resulting mixture is cultured in vitro as 37°C for 2 days. 0.2 ml of the culture medium is puls-labelled with 1 µCi of [3H]-thymidine for the last 20 hours and the [3H]-thymidine taken in the thymocytes is counted by β-scintillation counter.

The growth of 3T3 fibroblasts is examined in the following manner. Swiss Albino 3T3-L1 fibroblasts ($2.5 \times 10^4$ cells) ATCC CS-92.1 are cultured in a plastic titer well for 2 days at 37°C in a 5% $CO_2$-95% air environment in 0.9 ml of RPMI 1640 medium supplemented with 0.4% FCS plus 0.1 ml of active fraction of T cell activating factor(s) of CH Sephadex column, FGF from bovine pituitary gland (final concentration 20 ng/ml, Collaborative Research Inc., Mass., USA) or PBS. Cells in 10 areas randomly chosen are counted using a microscope and the total cell number per well is calculated.

The DNA synthesis of 3T3 fibroblasts is examined in the following manner. Swiss Albino 3T3-L1 fibroblasts are cultured under the same condition as in the exmination of the cell growth. After the cell culture for 16 hrs, 1 µC1 of [3H]-thymidine (5 Ci/mmol, Amersham, England) is added to the culture and labeled for 3 hrs. The cells are treated with 10% trichloroacetic acid (TCA), suspended and trapped on the GF/C membrane filter (Whatman Co.). Then, after washing the membrane with 5% TCA and ethanol, incorporated radio-activity is counted with Beckman scintillation counter.

Example 1

Production of T cell-activating factor by human fetal fibroblast cells.

Human fetal fibroblast cells were grown to a saturated cell concentration of about $10^6$ cells/ml according to the method of Reference Example 2. 50 ml of the cell culture was added to a medium which was prepared by adding 100 ng/ml (final concentration) TPA to MEM medium (product of Gibco) containing 10% FCS. Culturing was carried out at 37°C for 10 hours under aeration with 10% $CO_2$ and 90% air. The supernatant fluid was concentrated and fractionated as follows 25 ml of the supernatant fluid of the TPA treated culture medium was salted out with 60% $(NH_4)_2SO_4$ (pH 7.5) for concentration. The concentrate was centrifuged at 16,000 r.p.m. for 15 minutes. The precipitate was dissolved in 1.5 ml of 10 mM phosphate buffer with NaCl (pH 7.2) (PBS) and passed through a column ($1.8 \times 24$ cm) of Sephadex G-100 equilibrated with PBS. Elution was carried out with 10 mM PBS (pH 7.2) and the eluate was recovered in 2.5 ml fractions. T cell activities of the fractions were determined. The results are shown in Fig. 1. In the fractions of a molecular weight of 10,000 daltons and 30,000—40,000 daltons, activating substances were found. In Fig. 1, ●—● shows the presence of TPA and o—o shows the absence of TPA. The upper arrows a, b and c in Fig. 1 refer to bovine serum albumin [molecular weight 67 kilodaltons (referred to as Kd hereinafter)], horse-radish peroxides (molecular weight 40 Kd) and cytochrome C (molecular weight 13 Kd), respectively. The substances of a molecular weight of about 13,000 daltons and 30,000—40,000 daltons were treated with 25 µg/ml proteinase trypsin at 30°C for 30 minutes and subjected to fractionation with Sephadex G-100 column as mentioned above. Since the activity moved to lower molecular weight region it was clarified by the trypsin treatment that the substances fractionated in this Example contain proteins (see Table 1).

TABLE 1

| | [3H]-thymidine uptake (cpm) | | |
|---|---|---|---|
| Fraction No. | Control | TPA treated | TPA untreated |
| 5 | 4389±292 | — | — |
| 14 | 19631±624 | 5026±652 | 25.6 |
| 20 | 20291±809 | 6513±148 | 32.1 |

The activating factor of a molecular weight of 30,000 to 40,000 daltons is readily inactivated by heating and that of 10,000 daltons is heat resistant as shown in Table 1.

Table 1 shows the [3H]-thymidine uptake by the both factors after the treatment at 56°C for 30 minutes.

3

TABLE 2

[$^3$H]-thymidine uptake (cpm)

| Fraction No. | No treat-ment | Heat treatment | Heat Treatment /No treatment (%) |
|---|---|---|---|
| Control (PBS) | 3279±432 | — | — |
| 14 | 10592±163 | 5751±861 | 54.3 |
| 20 | 21724±582 | 19404±706 | 89.3 |

The same culturing as mentioned above was carried out except for the use of no serum or 1% serum. T cell-activating factor was not formed in the culture medium.

Example 2

The same procedures as in Example 1 were repeated except that fetal fibroblast cells infected with virus SV40 were used.

The infection of the fetal fibroblast cells with virus SV40 was carried out by infecting the cell lines established in Reference Example 2 with 100 virus particles per cell. The infected cells were maintained by the same method as for normal cells. The cells reached confluence in 4 days after inoculation into a four-fold diluted medium.

The same procedures as in Example 1 were repeated except that the thus infected fetal fibroblast cells were used to produce a T cell-activating factor of a molecular weight of about 10,000 daltons as illustrated in Fig. 2. Since the T cell-activating factor was inactivated by trypsin treatment and was stable to heat, it is likely to be the same as the T cell-activating factor of about 10,000 daltons produced in Example 1.

The T cell-activating factor obtained in this Example was heated and treated with 25 μg/ml trypsin. Then, 25 μg/ml soybean trypsin inhibitor (Seikagaku Kogyo Co., Ltd.) was added and the activity was measured. The results are shown in Table 3.

TABLE 3

| | [$^3$H]-thymidine uptake (cpm) |
|---|---|
| Control | 3218±403 |
| T cell-activating factor | 21620±521 |
| Heat treatment (56°C, 30 minutes) | 20713±685 |
| Trypsin treatment (25 μg/ml, 30°C, 60 minutes) | 5326±236 |

The T cell-activating factor of the present Example and fibroblast growth factor (referred to as FGF hereinafter) (product of Collaborative Research, U.S.A.) obtained from the bovine brain were examined for the effect on fibroblast cells.

That is, Swiss Albino 3T3-Ll fibroblasts (2.5×10$^4$ cells) ATCC CS-92.1 were cultured in a plastic titer well for 2 days at 37°C in a 5% CO$_2$-95% air environment in 0.9 ml of RPMI 1640 medium supplemented with 0.4% FCS plus 0.1 ml of active fraction of T cell-activating factor(s) or CM Sephadex column, FGF from bovine pituitary gland (Final concentration 20 ng/ml, Collaborative Research Inc., Mass., USA) or PBS. Cells in 10 areas randomly chosen were counted using a microscope and the total cell number per well was calculated.

TABLE 4

| | Cell number of fibroblasts/well (×10$^4$) | |
|---|---|---|
| Treatment | Experiment 1 | Experiment 2 |
| Control (PBS) | 2.7 | 2.5 |
| FGF (20 ng/ml) | 6.5 | 6.3 |
| Factor | 2.6 | 2.4 |

**0 108 919**

The T cell-activating factor obtained in this Example and FGF were examined for the activity of T cell activation (ConA depending T cell DNA synthetic activity). The results are shown in Table 5. 0.1 ml of the active fraction Nos. 20 and 21 of Sephadex G-100 column chromatography in Fig. 2 was used for the examination.

TABLE 5

| | $[^3H]$-thymidine uptake (cpm) |
|---|---|
| Control | $3528\pm516$ |
| FGF 5 ng/ml | $4320\pm214$ |
| 10 „ | $3276\pm329$ |
| 25 „ | $3926\pm682$ |
| 50 „ | $3682\pm915$ |
| T cell-activating factor | $20432\pm798$ |

The results show that the T cell-activating factor is different from FGF so far reported.

About 7.5 ml of 10 Kd factor from SV40-transformed human fibroblasts was recovered from the active fractions of Sephadex G-100 column chromatography and concentrated by salting out with 60% saturated ammonium sulfate (pH 7.5). After centrifugation, the precipitate was dissolved in 1.5 ml of 10 mM phosphate buffered saline (PBS, pH 7.4) and dialyzed against the same PBS. The sample was passed through a column of Concanavalin A Sepharose (Pharmacia, gel bed volume: 9 ml), which had been equilibrated with PBS, and eluted with PBS and PBS-containing 0.125 M α-methyl mannoside (α-mM) stepwise at 1.25 ml per fraction. As shown in Fig. 3, a part of the active substance was eluted with PBS only, but the remaining active substance was bound to Con A Sepharose and eluted with α-methyl mannoside-containing PBS. This result suggests that some portions of 10 Kd factor(s) produced by SV40-transformed human fibroblasts are glycoprotein.

Reference Example 1

Mouse 3T3 fibroblast cells were inoculated in RPMI1640 medium containing 100 µg/ml streptomycin, 100 units/ml penicillin and 5% FCS and culturing was carried out under aeration with 10% $CO_2$ and 90% air at 37°C for 3 days. By the cultivation, mouse 3T3 fibroblast cells reached a saturated cell concentration, so called confluent state.

Reference Example 2

Fetal human embryos of 2 months were treated with trypsin and cultured in Eagle's minimum medium [MEM, product of Nissui Seiyaku Co.) containing 10% PCS (product of Grand Island Biological Co., Gibco), 10 mM N-N-bis[2-hydroxyethyl]-2-aminoethane-sulfonic acid (BES), 5 mM N-tris[hydroxymethyl]-methyl-3-aminopropane-sulfonic acid (TES) and 5 mM N-hydroxymethyl-piperadine-N-2-ethanesulfonic acid (HEPES, product of Nakarai Kagaku Co.) under aeration with 10% $CO_2$ and 90% air at 37°C for 4 days. By the cultivation, human fibroblast cells reached a saturated cell concentration, so called confluent state.

Reference Example 3

T cell-activating factor produced by SV40-transformed fibroblasts is different from "tumor growth factors".

De Larco and Todaro reported that tumor virus-transformed mouse fibroblasts produce polypeptide growth factors that stimulate cell division in normal fibroblasts [Proc. Natl. Acad. Sci. USA (1978) 15, 40011, which are generally named "tumor growth factor". In order to examine whether T cell-activating factor of the present invention is derived from "tumor growth factor" or not, the active fraction at 10 Kd of Sephadex G-100 column in Fig. 2 was further fractionated by CM Sephadex column chromatography. As shown in Fig. 4, the active fractions of Sephadex G-100 column were concentrated by salting out with 60% saturated ammonium sulfate (pH 7.5). After centrifugation, the precipitate was dissolved in 1.5 ml of 10 mM Tris-HCl (pH 7.5) and dialyzed against 10 mM Tris-HCl (pH 7.5)−25 mM NaCl. The sample was passed through a CM Sephadex column (gel bed volume; 7.5 ml), which had been equilibrated with 10 mM Tris-HCl (pH 7.5)−25 mM NaCl, and eluted with 10 mM Tris-HCl (pH 7.5) containing 25 mM, 150 mM, 300 mM and 500 mM NaCl, stepwise at 1.5 ml per fraction. 0.1 ml of each fraction was examined for the DNA synthesis activities of T cells and 3T3 fibroblasts. As shown in Fig. 4, the major activity for T cell DNA synthesis was detected at 150 mM and the minor activity was detected at 500 mM NaCl (Open circles in the figure). On the other hand, DNA synthesis activities of 3T3 fibroblasts were observed at 25 mM and 300 mM NaCl, respectively

5

(triangles in the figure). This result suggests that T cell-activating factor produced by SV40-transformed human fibroblasts is different from "tumor growth factors".

Reference Example 4

The activities of stimulating factors from TPA-treated human fibroblasts are not derived from artifact ones by TPA.

Recently, Krakauer *et al.* claimed that TPA is bound to fetal calf serum proteins for the cell culture and the bound TPA shows a pseudo-activity of T cell DNA synthesis at high-molecular-weight fractions [J. Immunol. (1982) *129*, 939—941]. In order to study whether T cell-activating factors of the present invention are actually produced by TPA-treated human fibroblasts or not, the following control experiment was performed. The conditioned medium (culture liquor) of normal fibroblasts under a high serum condition (10% FCS) was mixed with TPA (100 ng/ml) and kept for 2 hrs at room temperatures. The mixture was concentrated by salting out with saturated ammonium sulfate (pH 7.5) (final concentration: 60%) as described above. After centrifugation, the precipitate was dissolved in 2 ml of 10 mM PBS and passed through Sephadex G-100 column (1.8×24 cm) by the same method as mentioned above.

The normal conditioned medium (culture liquor) and the fresh medium (RPMI 1640 medium-10% FCS) were treated by the same methods. As shown in Fig. 5, the activity at void volume of Sephadex G-100 column of TPA-containing mixture is higher than that of normal conditioned medium. This result suggests that the considerably high activity at void volume of the medium conditioned by TPA-treated fibroblasts in Fig. 5, seems to be an artifact activity by TPA. However, the activities of 30—40 Kd and 10 Kd factors of Sephadex G-100 column are not changed between the normal medium and TPA-containing mixture. This results suggests that the activities of 30—40 Kd and 10 Kd factors produced by TPA-treated fibroblasts are not artifact ones.

**Claims**

1. A process for producing a T cell-activating factor which comprises culturing human fibroblast cells in a medium containing a phorbol, lectin, entotoxin or a virus, accumulating the T cell-activating factor in the medium, and recovering the T cell-activating factor therefrom.

2. The process according to Claim 1, wherein the phorbol is phorbol, phorbol myristate, phorbol-13-acetate or phorbol myristic acetate (PMA).

3. The process according to Claim 1, wherein the virus is a cancer virus selected from SV40 and Kirsten murine sarcoma virus.

4. The process according to Claim 1, wherein the medium is selected from RPMI1640 medium containing fetal calf serum and MEM medium containing fetal calf serum.

5. The process according to Claim 4, wherein the concentration of the fetal calf serum is 5—10%.

**Patentansprüche**

1. Verfahren zur Herstellung eines T-Zellen aktivierenden Faktors, das Züchtung von Fibroblasten-Zellen des Menschen in einem ein Phorbol, Lectin, Endotoxin oder ein Virus enthaltendem Medium, Ansammlung des T-Zellen aktivierenden Faktors in dem Medium und Gewinnung des T-Zellen aktivierenden Faktors daraus umfaßt.

2. Verfahren nach Anspruch 1, wobei das Phorbol Phorbol, Phorbol-myristat, Phorbol-13-acetat oder Phorbol-myristinacetat (PMA) ist.

3. Verfahren nach Anspruch 1, wobei das Virus eines der Krebsviren SV40 oder Kirsten-Maus-Sarcom-Virus ist.

4. Verfahren nach Anspruch 1, wobei das Medium fötales Kälberserum enthaltendes RPMI1640 Medium oder fötales Kälberserum enthaltendes MEM-Medium ist.

5. Verfahren nach Anspruch 4, wobei die Konzentration des fötalen Kälberserums 5 bis 10% beträgt.

**Revendications**

1. Procédé de préparation d'un facteur activateur de cellules T nui consiste à cultiver des cellules fibroblastes humaines dans un milieu contenant un phorbol, une lectine, une endotoxine du un virus, à accumuler le facteur activateur de cellules T dans le milieu et à récupérer le facteur activateur de cellules T à partir de ce milieu.

2. Procédé selon la revendication 1, dans lequel le phorbol est le phorbol, le phorbol-myristate, le phorbol-13-acétate ou le phorbol-myristique-acétate (PMA).

3. Procédé selon la revendication 1, dans lequel le virus est un virus cancérigène choisi parmi le virus SV40 et le virus du sarcome murin de Kirsten.

4. Procédé selon la revendication 1, dans lequel le milieu est choisi parmi le milieu RPMI 1640 contenant du sérum foetal de veau et la milieu MEM contenant du sérum foetal de veau.

5. Procédé selon la revendication 4, dans lequel la concentration du sérum foetal de veau est comprise entre 5 et 10%.

Fig. 1

fraction No.

1

Fig. 2

Fig. 3

Fig. 4

Fig. 5